# EUROPEAN PATENT APPLICATION

(11) **EP 4 793 309 A1**
(43) Date of publication of application: **19.08.2026**
(21) Application number: 24885735.1
(22) Date of filing: 30.10.2024
(51) Int. Cl.: C08J 7/04, A61L 29/08, A61L 29/12

(54) **COATING FILM AND METHOD FOR FORMING COATING FILM**

(30) Priority: 01.11.2023 JP 2023187768
(71) Applicant: Terumo Kabushiki Kaisha, Tokyo 151-0072 (JP)
(72) Inventor: HATA, Mayu, Ashigarakami-gun, Kanagawa 259-0151 (JP)
(74) Representative: Prüfer & Partner mbB Patentanwälte · Rechtsanwälte
(86) International application number: PCT/JP2024/038611
(87) International publication number: WO 2025/094968

(57) **Abstract**

Provided are a coating film that satisfies conditions necessary for use in a light irradiation device, and a method for forming the coating film. A coating film 36 applied to a surface of a substrate, the coating film 36 including: a thermoplastic elastomer; and a pigment, in which a mass ratio between the thermoplastic elastomer and the pigment is less than 1:2.0, and, in the coating film 36, in a tensile test when a test piece acquired by applying the coating film 36 to the substrate having a width of 10 mm and a length of 40 mm is set in a tensile tester so that a grip distance is 25 mm, and is measured at a tensile speed of 25 mm/min, elongation when a test piece (A) including the substrate and the coating film 36 breaks is smaller than elongation when a test piece (B) including the substrate breaks, and the elongation of the test piece (A) is 40% or more of the elongation of the test piece (B).

## Description

### Technical Field

The present invention relates to a coating film applied to a surface of a substrate used in a medical light irradiation device and a method for forming the coating film.

### Background Art

Various medical devices that are each inserted into a living body for performing treatment, such as a catheter, are known. A medical device may require a coating film applied to a surface of a substrate as a marker for visually recognizing its position in a living body or a light-blocking body for preventing irradiation of light to an area outside a predetermined range in a light irradiation device.

As an example of a light irradiation device, a device that irradiates a lesion with light in photo-dynamic therapy (PDT) or photo-immunotherapy (PIT) performed using a photo-reactive substance having tumor cell selectivity is known. A medical light irradiation device includes at least a light irradiation unit that emits light and an elongated member having the light irradiation unit at a distal end portion. In this light irradiation device, a balloon is disposed at the distal end portion of the elongated member, a light irradiation body having the light irradiation unit is disposed inside the balloon, and light can be emitted from a light transmission window portion disposed on the balloon. The light irradiation device can maintain a constant distance between the lesion and the light irradiation unit by expanding the balloon, and can fix a position of the light irradiation unit with respect to the lesion, so that the lesion can be stably irradiated with light.

In order to form the light transmission window portion on the balloon, a light-blocking body formed from a coating film having low light transmittance is formed in a region other than the light transmission window portion on the surface of the balloon serving as a substrate. The coating film is formed by, for example, depositing metal on the substrate. As a light irradiation device having such a coating film, for example, there is a device described in Patent Literature 1.

### Citation List

### Patent Literature

Patent Literature 1: JP 2005-46640 A

### Summary of Invention

### Technical Problem

A coating film for a light irradiation device is not only required to have sufficiently low light transmittance, but also required to satisfy several conditions. The coating film is required to be low in cytotoxicity because the light irradiation device is inserted into a living body. Furthermore, the coating film is also required to have water resistance so as not to allow peeling off due to water to occur. Moreover, it is required that the coating film can be formed at a temperature at which the substrate is not deformed. In addition, in order to maintain flexibility of the substrate when the coating film is applied to the substrate, it is also required that the coating film follows deformation of the substrate when the substrate is elongated to a certain extent. A coating film satisfying these conditions and a method for forming the coating film have been demanded.

The present invention has been made to solve the above-described problems, and an object thereof is to provide a coating film that satisfies conditions necessary for use in a light irradiation device, and a method for forming the coating film.

### Solution to Problem

(1) A coating film according to the present invention, for achieving the above-described object, is a coating film applied to a surface of a substrate, the coating film including: a thermoplastic elastomer; and a pigment, in which a mass ratio between the thermoplastic elastomer and the pigment is less than 1:2.0, and, in the coating film, in a tensile test when a test piece acquired by applying the coating film to the substrate having a width of 10 mm and a length of 40 mm is set in a tensile tester so that a grip distance is 25 mm, and is measured at a tensile speed of 25 mm/min, elongation when a test piece (A) including the substrate and the coating film breaks is smaller than elongation when a test piece (B) including the substrate breaks, and the elongation of the test piece (A) is 40% or more of the elongation of the test piece (B).

(10) A method for forming a coating film, according to the present invention, for achieving the above-described object, includes: preparing a first solution in which, to make a mass ratio between a thermoplastic elastomer and a pigment less than 1:2.0, the pigment is dissolved in a solvent; preparing a second solution in which the thermoplastic elastomer is dissolved in the first solution; and applying and drying the second solution to a substrate, in which, in the coating film, in a tensile test when a test piece acquired by applying the coating film to the substrate having a width of 10 mm and a length of 40 mm is set in a tensile tester so that a grip distance is 25 mm, and is measured at a tensile speed of 25 mm/min, elongation when a test piece (A) including the substrate and the coating film breaks is smaller than elongation when a test piece (B) including the substrate breaks, and the elongation of the test piece (A) is 40% or more of the elongation of the test piece (B).

### Advantageous Effects of Invention

The coating film configured as described above has water resistance and can be formed at a temperature at which the substrate is not deformed. Furthermore, the coating film follows deformation of the substrate when the substrate is elongated to a certain extent, and can suppress peeling off or tearing from the substrate. From those described above, the coating film can satisfy characteristics necessary for use in a light irradiation device while having mechanical and optical characteristics for performing functions of the light irradiation device.

(2) In the coating film described above in (1), the elongation of the test piece (A) may be 90% or more of the elongation of the test piece (B). Accordingly, the coating film that can more securely follow the elongation of the substrate can reliably return to its original shape when the substrate is elongated and contracted.

(3) In the coating film described above in (1) or (2), the coating film may be, when evaluated in accordance with ISO10993, weaker in cell attribute intensity (IC₅₀ value) than a polyurethane film containing zinc dibutyldithiocarbamate (ZDBC) at a percentage of 0.25%, the polyurethane film serving as a positive control material B. Accordingly, the coating film can be weakened in cytotoxicity to an extent necessary for use in a light irradiation device.

(4) In the coating film described above in any one of (1) to (3), a film thickness of the coating film may be 60 µm or less. Accordingly, an outer diameter of a light irradiation device can be suppressed.

(5) In the coating film described above in any one of (1) to (4), a light transmittance of the coating film may be 31% or less. Accordingly, it is possible to satisfy optical performance as a coating film.

(6) In the coating film described above in any one of (1) to (5), the thermoplastic elastomer may be a polyurethane elastomer. Accordingly, the coating film can be enhanced in followability with respect to deformation of the substrate.

(7) In the coating film described above in any one of (1) to (6), the pigment may be titanium oxide, carbon black, or a mixture of the titanium oxide and the carbon black. Accordingly, the coating film may be white, black, or gray, and the coating film can satisfy requirements for cytotoxicity intensity.

(8) In the coating film described above in any one of (1) to (7), the substrate may be a balloon including a light transmission material, and a thickness of the balloon may be 30 µm. Accordingly, it is possible to satisfy biological safety and mechanical and optical characteristics of a balloon in a light irradiation device having a light irradiation body inside the balloon on which the coating film is formed so as to be configured to irradiate light in a certain direction.

(9) In the coating film described above in any one of (1) to (8), the mass ratio between the thermoplastic elastomer and the pigment may be 1:0.5 or less, a film thickness of the coating film may be 30 µm or less, a light transmittance of the coating film may be 0%, and the pigment may be carbon black. Accordingly, the coating film can be high in biological safety, and, furthermore, can be thinner in film thickness.

A method for forming the coating film configured as described above can form a coating film that has water resistance, can be formed at a temperature at which the substrate is not deformed, and satisfies characteristics necessary for a light irradiation device.

### Brief Description of Drawings

[Fig. 1] Fig. 1 is an overall view of a light irradiation device having a coating film according to a present embodiment and an endoscope device.
[Fig. 2] Fig. 2 is a perspective view of a balloon.
[Fig. 3] Fig. 3 is an enlarged cross-sectional view of an area around a distal end portion of the light irradiation device inserted into the endoscope device.

### Description of Embodiments

Hereinafter, an embodiment of the present invention will be described with reference to the drawings. Note that, the dimensions of the drawings may be exaggerated and different from actual dimensions for convenience of description in some cases. In the present specification and the drawings, components having substantially the same functional configuration are denoted by the same reference numerals, and the redundant description will not be repeated. In the present specification, a side of a device to be inserted into a living body is referred to as a "distal side", and a side to be operated is referred to as a "proximal side".

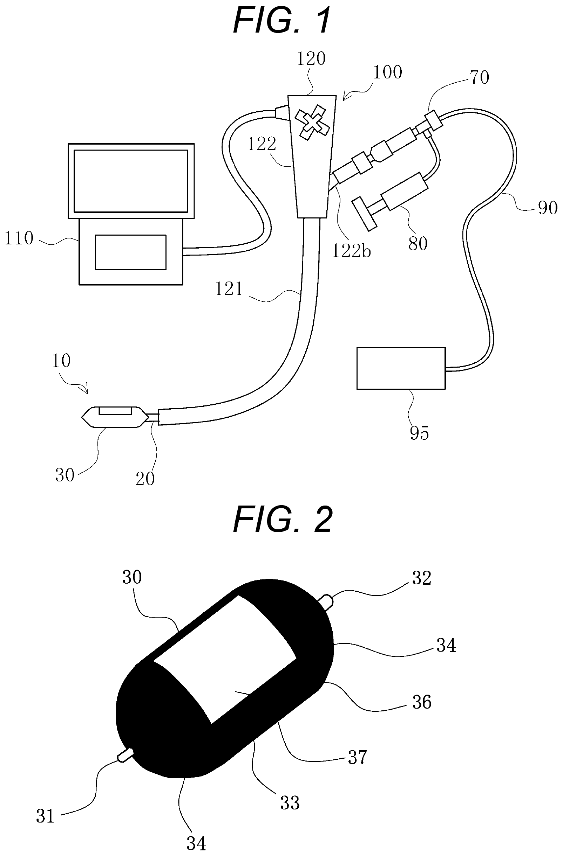

A light irradiation device 10 according to the present embodiment can be configured to be compatible with treatment of approaching tumor cells as an endoscope system via an endoscope device 100. As illustrated in Fig. 1, the light irradiation device 10 is used by being inserted into the endoscope device 100, for example. A type of the tumor is not particularly limited, but for example, in a case where the light irradiation device 10 is inserted into the endoscope 100 and used as an endoscope system, the light irradiation device 10 can be applied to a tumor generated in a hollow organ such as the esophagus, the stomach, the small intestine, the large intestine, the urinary tract, a blood vessel, the ear canal, the auditory tube, and the nasal cavity. The light irradiation device 10 in the present embodiment is used, for example, in photo-immunotherapy taught in Japanese Patent No. 6127045 in which a drug adsorbed to a target cell is irradiated with light to destroy the target cell. The target cell is a tumor cell such as a cancer cell or a cell of a precancerous lesion. In this therapeutic method, a photosensitizer in which an antibody that specifically binds to only a specific antigen on the surface of a tumor cell and a photosensitizer paired with the antibody are adsorbed is used as a drug. The antibody is not particularly limited, and examples thereof include panitumumab, trastuzumab, HuJ591, pertuzumab, lapatinib, palbociclib, olaparib, and the like. The photosensitizer is, for example, but not limited to, hydrophilic phthalocyanine, which is a substance reactive to near infrared rays having a wavelength of about 700 nm (IR700). When IR700 receives near infrared rays having a wavelength falling within a range of about 660 to about 740 nm, a ligand of a functional group that ensures water solubility is broken, and a structural change from water solubility to hydrophobicity occurs. The membrane protein is extracted by this structural change, a hole is formed in the cell membrane, and water enters the cell, whereby the tumor cell can be ruptured and destroyed. In addition, the IR700 is excited by receiving near infrared rays and emits fluorescence having a wavelength different from the excitation wavelength. For example, when the IR700 is excited by receiving near infrared rays having a wavelength around 690 nm, the IR700 emits fluorescence having a wavelength around 700 nm. The IR700 undergoes a structural change while emitting fluorescence by photoreaction, and does not perform emission of fluorescence after it destroys tumor cells and serves as a drug.

The endoscope device 100 includes an endoscope main body 120 connected to a control display portion 110. An operator inserts the endoscope main body 120 into a living body and performs various kinds of operation. The endoscope main body 120 includes a long insertion portion 121 to be inserted into the living body and a handle portion 122 disposed at a proximal end portion of the long insertion portion 121. The handle portion 122 has a forceps port 122b communicating with a lumen 125 that the long insertion portion 121 has. The light irradiation device 10 is inserted into the endoscope device 100 from the forceps port 122b of the handle portion 122.

The light irradiation device 10 includes an elongated shaft portion 20 inserted into the endoscope device 100 and a balloon 30 disposed at a distal end portion of the shaft portion 20 and exposed to a distal end side of the elongated insertion portion 121. Moreover, the light irradiation device 10 includes a proximal end hub 70 disposed at a proximal end portion of the light irradiation device 10. An expansion device 80 for injecting a fluid for expanding the balloon 30 is connected to the proximal end hub 70. As the expansion device 80, for example, an in-deflator can be used.

A light irradiation body 90 that emits light in the balloon 30 is inserted into the light irradiation device 10. The light irradiation body 90 is exposed closer to a proximal side of the light irradiation device 10 than the proximal end hub 70 and is connected to a light source portion 95 that outputs light. The light irradiation body 90 includes a light irradiation unit 92 disposed on a distal side in a long axis direction of the light irradiation body 90 and an optical fiber connected to the light irradiation unit 92 and extending toward a proximal side in the long axis direction of the light irradiation device 90. For example, the light irradiation body 90 includes a side emission fiber configured to emit light in an entire-circumference manner outwardly in radial directions at a distal end portion. Note that the light irradiation unit 92 and the optical fiber can be configured so that a diameter of the light irradiation unit 92 is larger than a diameter of the optical fiber in the long axis direction of the light irradiation body 90.

As illustrated in Fig. 2, the balloon 30 has a portion between a distal-most end 31 and a proximal-most end 32, which can expand in the radial directions. A central portion in the long axis direction of the balloon 30 is a straight portion 33 that is constant in diameter in the long axis direction, and both end portions in the long axis direction of the balloon 30 are tapered portions 34 having diameters decreasing toward both ends. As illustrated in Fig. 2, the tapered portions 34 can be formed in a hemispherical shape. Note that the tapered portions 34 may have a conical shape. The balloon 30 is formed from a light transmission material such as nylon or urethane. A thickness of the balloon 30 is, for example, 30 µm.

The light irradiation body 90 is disposed inside the balloon 30. The balloon 30 includes a light transmission window portion 37 that allows light to transmit from the light irradiation unit 92 included in the light irradiation body 90, and is coated with a coating film 36 having a lower transmittance of light from the light irradiation unit 92 than a transmittance of the light transmission window portion 37. The coating film 36 is formed by being applied to an outer surface of the balloon using the balloon 30 as a substrate. In other words, the balloon 30 includes, as a light-blocking body, a film body formed from the coating film 36 coated on the outer surface of the balloon 30. A surface of the balloon 30 serving as the substrate is not covered with the coating film 36, and thus the light transmission window portion 37 can allow light to transmit from the light irradiation unit 92. That is, a portion not covered with the light-blocking body is defined as the light transmission window portion 37. The light transmission window portion 37 may be disposed wholly in a circumferential direction (360 degrees) or may be disposed partially in the circumferential direction (for example, 180 degrees).

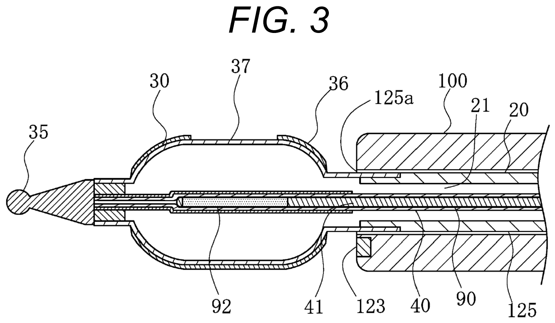

As illustrated in Fig. 3, the balloon 30 can be expanded by being exposed to a distal side from a distal opening 125a of the lumen 125 that the endoscope device 100 has. The endoscope device 100 includes an endoscope scope 123 at its distal end portion, allowing a state of the balloon 30 to be visually recognized. A proximal end portion of the balloon 30 is bonded to the distal end portion of the shaft portion 20 inserted into the lumen 125 of the endoscope device 100. A distal end tip 35 is disposed at a distal end portion of the balloon 30.

A tubular body 40 having a lumen 41 running in the long axis direction is disposed inside the balloon 30. The tubular body 40 extends in the long axis direction inside the shaft portion 20. A portion inside the shaft portion 20 and outside the tubular body 40 is an expansion lumen 21 through which the fluid for expanding the balloon 30 flows.

The light irradiation body 90 is inserted into the lumen 41 of the tubular body 40. The light irradiation body 90 that is the side emission fiber configured to emit light in an entire circumference manner outwardly in the radial directions has the light irradiation unit 92 configured to emit light in the radial directions at the distal end portion. The tubular body 40 is formed of a light transmission material in order to allow light to transmit from the light irradiation unit 92.

Light from the light irradiation unit 92 passes through and exits the light transmission window portion 37 of the balloon 30. A portion other than the light transmission window portion 37 of the balloon 30 is covered with the coating film 36, which is low in transmittance of light from the light irradiation unit 92. Since only a lesion and a region around the lesion are selectively irradiated with light from the light irradiation unit 92, one optical characteristic required for the coating film 36 is sufficiently low light transmittance. Furthermore, mechanical characteristics required for the coating film 36 are flexibility in deformation for following expansion and contraction of the balloon 30, and water resistance and rubbing resistance when the balloon 30 is inserted into a living body. In addition to these requirements, it is also necessary that the coating film 36 that is used for the light irradiation device 10 that is to be inserted into a living body has biological safety.

The coating film 36 formed on the balloon 30 serving as the substrate will now be described herein in detail. The coating film 36 includes a thermoplastic elastomer and a pigment. In the present embodiment, a polyurethane elastomer is used as the thermoplastic elastomer. As the thermoplastic elastomer, a polystyrene elastomer, a polyamide elastomer, or the like can also be used, and the thermoplastic elastomer is not limited to the polyurethane elastomer.

The pigment is not particularly limited as long as it can color the coating film 36, but, in the present embodiment, titanium oxide is used as a pigment for white coloring, and carbon black is used as a pigment for black coloring. Furthermore, the coating film 36 can also be colored gray by mixing titanium oxide and carbon black. The pigment may be other than those described above, and, for example, phthalocyanine blue, phthalocyanine green, or the like can also be used.

As examples of the coating films 36, the coating films 36 were formed on substrates respectively with compositions and thicknesses listed in Table 1. Note that a transmittance marked with * was a transmittance estimated from a measured reflectance.

**[Table 1]**

| | Mass ratio between thermoplastic elastomer and pigment | Film thickness of coating film | Light transmittance |
|---|---|---|---|
| Titanium oxide | 1:1.0 | 15 µm | 31%* |
| | 1:1.0 | 30 µm | 20%* |
| | 1:1.0 | 40 µm | 15% |
| | 1:1.1 | 65 µm | 9%* |
| | 1:1.2 | 60 µm | 9%* |
| | 1:1.3 | 90 µm | 7%* |
| | 1:1.4 | 85 µm | 8%* |
| | 1:1.5 | 14 µm | 27%* |
| | 1:2.0 | - | - |
| Titanium oxide + carbon black | 1:1.0 | 12 µm | 8% |
| Carbon black | 1:0.5 | 6 µm | 1% |
| | 1:0.4 | 7 µm | 0% |
| | 1:0.5 | 8 µm | 0% |
| | 1:0.5 | 20 µm | 0% |

A method for forming each of the coating films 36 is as follows. A thermoplastic elastomer and a pigment, which are to be materials of the coating film 36, and a solvent for dissolving the thermoplastic elastomer and the pigment are first prepared. The pigment is first dissolved in the solvent to prepare a first solution. Next, the thermoplastic elastomer is dissolved in the solution (first solution) acquired by dissolving the pigment in the solvent to prepare a second solution. As described above, by first dissolving the pigment in the solvent and then dissolving the thermoplastic elastomer in the solvent, the pigment can be easily diffused into the solvent. As the solvent, THF (tetrahydrofuran) can be used. As illustrated in Table 1, a mass ratio between the thermoplastic elastomer and the pigment was set in a range of 1:1.0 to 1:2.0 when the pigment was titanium oxide, and in a range of 1:0.4 to 1:0.5 when the pigment was carbon black. Furthermore, when titanium oxide and carbon black were mixed, the mass ratio of the thermoplastic elastomer and the pigment was set to 1:1.0. A mixing ratio between titanium oxide and carbon black was 100:1. The thermoplastic elastomer and the pigment were dissolved in the solvent, and the solution was prepared. For a proportion of the solution, when the mass ratio between the thermoplastic elastomer and the pigment was 1:1.0, the thermoplastic elastomer was 10 mass%, the pigment was 10 mass%, and the solvent was 80 mass%.

Once the solution was prepared, the solution was applied to the substrate. The substrate for each of the coating films 36 in Table 1 was a PET film. The solution was applied through dipping. A film thickness of each of the coating films 36 could be adjusted with a pulling speed and a number of times of dipping. The pulling speed of dipping was, for example, 0.5 mm/s, and the number of times of dipping was, for example, 1.

After the solution was applied to the substrate, the coating film 36 was dried. THF serving as the solvent has a boiling point of 66°C, and can be dried even at a room temperature. The solvent for forming the coating film 36 may be other than THF, and may be a desired solvent as long as it can be dried at a low temperature equal to or higher than the room temperature and equal to or lower than 60°C. Accordingly, the coating film 36 could be formed without deforming the substrate. Specific examples of the solvents may include methanol and hexane in addition to THF.

When the coating films 36 were formed under the conditions illustrated in Table 1, powder was generated on the surfaces, and the coating films 36 were not sufficiently formed, in a case where the mass ratio between the thermoplastic elastomer and the pigment was 1:2.0, and, therefore, the mass ratio between the thermoplastic elastomer and the pigment for forming each of the coating films 36 needs to be less than 1:2.0.

It is desirable that the film thickness of each of the coating films 36 is as small as possible while satisfying certain optical characteristics in order to suppress an outer diameter of the light irradiation device 10. Under the conditions illustrated in Table 1, and when the pigment is carbon black, the coating films 36 could have a sufficiently low value of transmittance when the film thicknesses were 30 µm or less. Furthermore, the light transmittance was 9% when the pigment was titanium oxide, the mass ratio between the thermoplastic elastomer and the pigment was 1:1.2, and the film thickness of the coating film 36 was 60 µm, the light transmittance was 31% when the mass ratio between the thermoplastic elastomer and the pigment was 1:1.0 and the film thickness was 15 µm, and the light transmittance was 27% when the mass ratio between the thermoplastic elastomer and the pigment was 1:1.5 and the film thickness was 14 µm. Furthermore, the light transmittance was 8% when the pigment was a mixture of titanium oxide and carbon black, the mass ratio between the thermoplastic elastomer and the pigment was 1:1.0 and the film thickness was 12 µm. From these results, by setting the film thickness of the coating film 36 to 60 µm or less, at least the light transmittance can be set to 31% or less, and the optical characteristics as the coating film 36 can be sufficiently secured. Furthermore, by setting the film thickness of the coating film 36 to 30 µm or less (more preferably 22 µm or less), the coating film can follow expansion and contraction of the balloon 30 serving as the substrate and can deform, and the mechanical characteristics of the balloon 30 such as water resistance, rubbing resistance, and insertion and removal easiness when the balloon 30 is inserted into a forceps channel of an endoscope or into a living body can be sufficiently secured.

The coating film 36 needs to be weak in cytotoxicity in order to secure the above-described biological safety. Intensity of cytotoxicity can be assessed using an IC₅₀ value. The IC₅₀ value is a concentration (%) of a test solution that inhibits a colony formation rate (an average value of a number of colonies in a control group or a solvent control group is taken as 100%) by 50%. In the present embodiment, evaluation was performed in accordance with ISO 10993-5 (2009); Biological evaluation of medical devices - Part 5: Tests for in vitro cytotoxicity.
Titanium oxide used in the coating films 36 illustrated in Table 1 each had an IC₅₀ value ranging from 78 to 100%. Furthermore, since carbon black has no cytotoxicity, the IC₅₀ value was 100% or more. On the other hand, in the present embodiment, an IC₅₀ value of a positive control material B (polyurethane film containing zinc dibutyldithiocarbamate (ZDBC) at a percentage of 0.25%) was 60%. It was confirmed that all of the coating films 36 illustrated in Table 1, each of which had a cytotoxicity intensity (IC₅₀ value) of 60% or more, were weaker in cytotoxicity than the polyurethane film containing zinc dibutyldithiocarbamate (ZDBC) at a percentage of 0.25%, which served as the positive control material B.

The coating film 36 according to the present embodiment, which contains the thermoplastic elastomer, has flexibility and can expand and contract in accordance with expansion and contraction of the balloon 30. That is, the coating film 36 has followability to deformation of the balloon 30 serving as the substrate. Furthermore, the coating film 36 has high adhesiveness to the substrate, and can have high abrasion resistance in a living body. Furthermore, the coating film 36 that contains the thermoplastic elastomer has water resistance. Therefore, the coating film 36 can satisfy the above-described mechanical characteristics.

Tensile tests were performed on substrates each having the coating film 36 and substrates each having no coating film 36. Test pieces (A) in each of which the coating film 36 was applied to the substrate having a width of 10 mm and a length of 40 mm and test pieces (B) each formed only of the substrate having a width of 10 mm and a length of 40 mm were first prepared. These test pieces were each set in a tensile tester so that a grip distance was 25 mm, and measurement was performed at a tensile speed of 25 mm/min.

Results of the tensile tests for the three test pieces (A) and the three test pieces (B) are illustrated in Table 2.

**[Table 2]**

| Test piece (A) | 1 | 2 | 3 |
|---|---|---|---|
| Maximum test force (N) | 32.104 | 37.118 | 37.159 |
| Increase in grip distance (mm) | 28.785 | 27.664 | 27.848 |
| | | | |

| Test piece (B) | 1 | 2 | 3 |
|---|---|---|---|
| Maximum test force (N) | 32.704 | 33.068 | 29.825 |
| Increase in grip distance (mm) | 29.437 | 29.018 | 28.256 |

As a result of the tensile tests, an average value of elongation when each of the test pieces (A) each formed of the substrate and the coating film 36 breaks is 28.099 mm, whereas an average value of elongation when each of the test pieces (B) each formed only of the substrate breaks is 28.904 mm. That is, the test pieces (A) each formed of the substrate and the coating film 36 are each less in elongation when broken, compared with the test pieces (B) each formed of the substrate. Note herein that elongation refers to an elongation percentage of a balloon at each pressure, and is expressed as a percentage. Furthermore, elongation when broken refers to an elongation percentage when a test piece is broken, and is expressed as a percentage. That is, elongation is acquired by dividing a value acquired by subtracting the grip distance of the test piece before tension is applied from the grip distance of the test piece when broken by the grip distance of the test piece before tension is applied and by multiplying the resultant value by 100.

A ratio between smallest elongation when each of the test pieces (A) broke, which was 27.664 mm/25.000 mm × 100, and largest elongation when each of the test pieces (B) broke, which was 29.437 mm/25.000 mm × 100, was 93.98% (about 94%). Therefore, an elongation amount when each of the test pieces (A) broke with respect to the length of the grip distance before tension is applied was in a range of 90% or more of an elongation amount when each of the test pieces (B) broke. Therefore, the elongation when each of the test pieces (A) broke was in a range of 90% or more of the elongation when each of the test pieces (B) broke. When the coating film 36 described above is formed when a balloon is used as a substrate, and when the balloon is used and expanded and contracted in a living body, the coating film 36 follows elongation of the balloon due to expansion and elongates, and also follows contraction of the balloon when the balloon is contracted and removed from an interior of the living body, preventing an operation of folding the balloon from being hindered.

In order to confirm the mechanical properties of substrates, tests described below were performed. As the substrates, balloons made of nylon 12 were prepared. As the balloons made of the nylon 12, a balloon D having an outer diameter of 20 mm and a length of 45 mm and a balloon E having an outer diameter of 15 mm and a length of 45 mm were prepared. Both the balloon D and the balloon E were uncoated balloons that were not coated with the coating film 36. For the balloon D and the balloon E, the balloons were each expanded using an in-deflator at a pressure increment of 0.5 atm and elongation was measured. Note herein that elongation refers to an elongation percentage of a balloon at each pressure, and is expressed as a percentage. Furthermore, elongation when broken refers to an elongation percentage when the balloon is broken (exploded), and is expressed as a percentage. That is, by assuming that the outer diameter of the balloon (balloon before tension is applied) at atmospheric pressure (1.0 atm) is 0% serving as a reference, a degree of elongation until the balloon is exploded is expressed. Note herein that the balloon D and the balloon E were designed to have a rated diameter (outer diameter) of about 20 mm and about 15 mm at 2.0 atm, respectively.

For example, and in one example, the balloon D had a diameter of 18.467 mm at the atmospheric pressure (1.0 atm) and a diameter of 19.787 mm at normal use pressure (2.0 atm). The balloon D was expanded to a diameter of 21.567 mm at 5.0 atm, but exploded at 5.5 atm. Therefore, a maximum expansion diameter of the balloon D was 21.567 mm, and the balloon D was elongated by 16.79% based on the diameter at the atmospheric pressure. Furthermore, the balloon D was elongated by 7.15% at the normal use pressure based on the diameter at the atmospheric pressure. Therefore, it can be seen that a ratio between the elongation at the normal use pressure and the elongation at the maximum expansion diameter of the balloon D was about 42.58%. In one example, the balloon E had a diameter of 14.696 mm at the atmospheric pressure (1.0 atm) and a diameter of 15.533 mm at the normal use pressure (2.0 atm). The balloon E was expanded to a diameter of 17.475 mm at 8.0 atm, but exploded at 8.5 atm. Therefore, a maximum expansion diameter of the balloon E was 17.475 mm, and the balloon E was elongated by 18.91% based on the diameter at the atmospheric pressure. Furthermore, the balloon E was elongated by 5.70% at the normal use pressure based on the diameter at the atmospheric pressure. Therefore, it can be seen that a ratio between the elongation at the normal use pressure and the elongation at the maximum expansion diameter of the balloon E was about 30.14%. From those described above, it was found that the balloon having no coating film 36 was elongated by 30% or more of the maximum expansion diameter at the normal use pressure. It is required that the coating film 36 follows deformation of a balloon serving as a substrate, and neither peeling off nor tearing on the balloon occurs. Therefore, the substrate having the coating film 36 is required to follow an elongation of 30% or more. On the other hand, due to the applied coating film 36, elongation when the substrate having the coating film 36 breaks is smaller than that of the substrate having no coating film 36. Thus, it can be said that the substrate having the coating film 36 is smaller in elongation when broken with respect to the outer diameter of the original balloon (the diameter (outer diameter) of the balloon at the atmospheric pressure) than the substrate having no coating film 36, and elongation of the substrate having the coating film 36 with respect to the outer diameter of the original balloon may be 30% or more of elongation of the substrate having no coating film 36 when broken with respect to the outer diameter of the balloon before tension is applied. In fact, in a balloon F that is made of the nylon 12, has the coating film 36, and has an outer diameter of 15 mm and a length of 45 mm, elongation at the normal use pressure was 40% or more of elongation of the balloon E at the maximum expansion diameter. More specifically, the balloon F had a diameter of 13.679 mm at the atmospheric pressure (1.0 atm) and a diameter of 14.737 mm at the normal use pressure (2.0 atm). The balloon F was expanded to a diameter of 17.152 mm at 7.0 atm, but exploded at 7.5 atm. The balloon F was elongated by 7.73% at the normal use pressure based on the diameter at the atmospheric pressure. Therefore, it can be seen that a ratio between the elongation of the balloon F at the normal use pressure and the elongation of the balloon E at the maximum expansion diameter was about 40.90%. Therefore, it can be said that elongation when a substrate having the coating film 36 breaks with respect to an outer diameter of an original balloon may be 40% or more of elongation when a substrate having no coating film 36 breaks with respect to an outer diameter of an original balloon. According to the configuration described above, in the light irradiation device 10 including the balloon 30, the coating film 36 can effectively function as a light-blocking body when the balloon 30 is disposed in a living body and expanded.

As described above, (1) the coating film 36 according to the present embodiment is a coating film 36 applied to a surface of a substrate, the coating film 36 including: a thermoplastic elastomer; and a pigment, in which a mass ratio between the thermoplastic elastomer and the pigment is less than 1:2.0, and, in the coating film 36, in a tensile test when a test piece acquired by applying the coating film 36 to the substrate having a width of 10 mm and a length of 40 mm is set in a tensile tester so that a grip distance is 25 mm, and is measured at a tensile speed of 25 mm/min, elongation when a test piece (A) including the substrate and the coating film 36 breaks is smaller than elongation when a test piece (B) including the substrate breaks, and the elongation of the test piece (A) is 40% or more of the elongation of the test piece (B). The coating film 36 configured as described above has water resistance and can be formed at a temperature at which the substrate is not deformed. Furthermore, the coating film follows deformation of the substrate when the substrate is elongated to a certain extent, and can suppress peeling off or tearing from the substrate. From those described above, the coating film 36 can satisfy characteristics necessary for use in the light irradiation device 10 while having mechanical and optical characteristics for performing functions of the light irradiation device 10.

(2) In the coating film 36 described above in (1), the elongation when the test piece (A) breaks may be 93.5% or more of the elongation when the test piece (B) breaks. Accordingly, the coating film 36 that can more securely follow the elongation of the substrate can reliably return to its original shape when the substrate is elongated and contracted.

(3) In the coating film 36 described above in (1) or (2), the coating film 36 may be, when evaluated in accordance with ISO10993, weaker in cell attribute intensity (IC₅₀ value) than a polyurethane film containing zinc dibutyldithiocarbamate (ZDBC) at a percentage of 0.25%, the polyurethane film serving as a positive control material B. Accordingly, the coating film 36 can be weakened in cytotoxicity to an extent necessary for use in the light irradiation device 10.

(4) In the coating film 36 described above in any one of (1) to (3), a film thickness of the coating film 36 may be 60 µm or less. Accordingly, an outer diameter of a light irradiation device can be suppressed.

(5) In the coating film 36 described above in any one of (1) to (4), a light transmittance of the coating film 36 may be 31% or less. Accordingly, it is possible to satisfy optical performance when used as the coating film 36.

(6) In the coating film 36 described above in any one of (1) to (5), the thermoplastic elastomer may be a polyurethane elastomer. Accordingly, the coating film 36 can be enhanced in followability with respect to deformation of the substrate.

(7) In the coating film 36 described above in any one of (1) to (6), the pigment may be titanium oxide, carbon black, or a mixture of the titanium oxide and the carbon black. Accordingly, the coating film 36 may be white, black, or gray, and the coating film 36 can satisfy requirements for cytotoxicity intensity.

(8) In the coating film described above in any one of (1) to (7), the substrate may be the balloon 30 including a light transmission material, and a thickness of the balloon 30 may be 30 µm. Accordingly, it is possible to satisfy biological safety and mechanical and optical characteristics of the balloon 30 in the light irradiation device 10 having the light irradiation body 90 inside the balloon 30 on which the coating film 36 is formed so as to be configured to irradiate light in a certain direction.

(9) In the coating film 36 described above in any one of (1) to (8), the mass ratio between the thermoplastic elastomer and the pigment may be 1:0.5 or less, a film thickness of the coating film 36 may be 30 µm or less, a light transmittance of the coating film 36 may be 0%, and the pigment may be carbon black. Accordingly, the coating film 36 can be high in biological safety, and, furthermore, can be thinner in film thickness.

(10) A method for forming the coating film 36 according to the present embodiment, includes: preparing a first solution in which, to make a mass ratio between a thermoplastic elastomer and a pigment less than 1:2.0, the pigment is dissolved in a solvent; preparing a second solution in which the thermoplastic elastomer is dissolved in the first solution; and applying and drying the second solution to a substrate, in which, in the coating film 36, in a tensile test when a test piece acquired by applying the coating film 36 to the substrate having a width of 10 mm and a length of 40 mm is set in a tensile tester so that a grip distance is 25 mm, and is measured at a tensile speed of 25 mm/min, elongation when a test piece (A) including the substrate and the coating film 36 breaks is smaller than elongation when a test piece (B) including the substrate breaks, and the elongation of the test piece (A) is 40% or more of the elongation of the test piece (B). The method for forming the coating film 36 configured as described above can form the coating film 36 that has water resistance, can be formed at a temperature at which the substrate is not deformed, and satisfies characteristics necessary for a light irradiation device.

Note that the present invention is not limited to the embodiment described above, and those skilled in the art can make various modifications within the technical idea of the present invention.

Note that the present application is based on Japanese Patent Application No. 2023-187768 filed on November 1, 2023, the entire disclosed content of which is incorporated herein by reference.

### Reference Signs List

- 10: Light irradiation device
- 20: Shaft portion
- 21: Expansion lumen
- 30: Balloon
- 36: Light-blocking body
- 37: Light transmission window portion
- 40: Tubular body
- 41: Lumen
- 100: Endoscope device
- 122: Handle portion
- 125: Lumen

## Claims

1. A coating film applied to a surface of a substrate, the coating film comprising:
a thermoplastic elastomer; and a pigment,
wherein a mass ratio between the thermoplastic elastomer and the pigment is less than 1:2.0, and,
in the coating film, in a tensile test when a test piece acquired by applying the coating film to the substrate having a width of 10 mm and a length of 40 mm is set in a tensile tester so that a grip distance is 25 mm, and is measured at a tensile speed of 25 mm/min, elongation when a test piece (A) including the substrate and the coating film breaks is smaller than elongation when a test piece (B) including the substrate breaks, and the elongation of the test piece (A) is 40% or more of the elongation of the test piece (B).

2. The coating film according to claim 1, wherein the elongation of the test piece (A) is 90% or more of the elongation of the test piece (B).

3. The coating film according to claim 1 or 2, wherein the coating film is, when evaluated in accordance with ISO10993, weaker in cell attribute intensity (IC₅₀ value) than a polyurethane film containing zinc dibutyldithiocarbamate (ZDBC) at a percentage of 0.25%, the polyurethane film serving as a positive control material B.

4. The coating film according to claim 1 or 2, wherein a film thickness of the coating film is 60 µm or less.

5. The coating film according to claim 1 or 2, wherein a light transmittance of the coating film is 31% or less.

6. The coating film according to claim 1 or 2, wherein the thermoplastic elastomer is a polyurethane elastomer.

7. The coating film according to claim 1 or 2, wherein the pigment is titanium oxide, carbon black, or a mixture of the titanium oxide and the carbon black.

8. The coating film according to claim 1 or 2, wherein the substrate is a balloon including a light transmission material, and a thickness of the balloon is 30 µm.

9. The coating film according to claim 1 or 2, wherein the mass ratio between the thermoplastic elastomer and the pigment is 1:0.5 or less, a film thickness of the coating film is 30 µm or less, a light transmittance of the coating film is 0%, and the pigment is carbon black.

10. A method for forming a coating film, comprising:
preparing a first solution in which, to make a mass ratio between a thermoplastic elastomer and a pigment less than 1:2.0, the pigment is dissolved in a solvent;
preparing a second solution in which the thermoplastic elastomer is dissolved in the first solution; and
applying and drying the second solution to a substrate,
wherein, in the coating film, in a tensile test when a test piece acquired by applying the coating film to the substrate having a width of 10 mm and a length of 40 mm is set in a tensile tester so that a grip distance is 25 mm, and is measured at a tensile speed of 25 mm/min, elongation when a test piece (A) including the substrate and the coating film breaks is smaller than elongation when a test piece (B) including the substrate breaks, and the elongation of the test piece (A) is 40% or more of the elongation of the test piece (B).
